# FASCICULE DE BREVET EUROPEEN

(11) **EP 1 131 050 B1**
(45) Date de publication et mention de la délivrance du brevet: **26.05.2004**
(21) Numéro de dépôt: 00964295.0
(22) Date de dépôt: 15.09.2000
(51) Int. Cl.: A61K 9/00, A61K 9/20

(54) **FORMES GALENIQUES A DELITEMENT RAPIDE EN BOUCHE ET LEUR PROCEDE DE PREPARATION**
ORALE ARZNEIFORMEN MIT SCHNELLER AUFLÖSBARKEIT IM MUND UND VERFAHREN ZU IHRER HERSTELLUNG
GALENIC FORMULATIONS FAST DISINTEGRATING IN THE MOUTH AND METHOD FOR PREPARING SAME

(30) Priorité: 15.09.1999 FR 9911513
(43) Date de publication de la demande: 12.09.2001
(73) Titulaire: CLL Pharma, Arenas, 06200 Nice (FR)
(72) Inventeur: LARUELLE, Claude, F-06270 Villeneuve-Loubet (FR); ZAKARIAN, No[l, F-13009 Marseille (FR); GIMET, René, F-06560 Valbonne (FR); TOSELLI, Dominique, F-06000 Nice (FR)
(74) Mandataire: Vialle-Presles, Marie José
(86) Numéro de dépôt international: PCT/FR2000/002563
(87) Numéro de publication internationale: WO 2001/019336

(56) Documents cités:
- EP-A- 0 376 891
- WO-A-97/28788
- WO-A-99/47126

## Description

La présente Invention se rapporte à des formes galéniques à délitement rapide en bouche et à leur procédé de préparation.

En thérapeutique, la simplicité de l'administration orale d'un principe actif médicamenteux a toujours été considérée comme un avantage majeur, comme en témoigne la grande diversité des formes galéniques destinées à cette voie d'administration (comprimés nus, enrobés et effervescents, gélules, capsules molles, solutés à dissoudre, suspensions buvables prêtes à l'emploi, ...).

S'agissant des formes solides, celles-ci présentent toutefois deux inconvénients. D'une part, elles nécessitent la prise associée d'un liquide propre à faciliter leur déglutition. Or, il existe de nombreuses situations dans lesquelles il peut être appréciable de pouvoir prendre un médicament par voie orale dont l'ingestion ne nécessite pas d'absorber simultanément un liquide. D'autre part, certains patients et, notamment, les enfants et les personnes âgées, connaissent des difficultés de déglutition telles qu'il leur est difficile et, par conséquent, désagréable, d'ingérer une forme galénique solide, même en présence d'un liquide.

Quant aux formes liquides, elles se présentent généralement dans des conditionnements volumineux ou fragiles (flacons, ampoules en verre), ce qui les rend peu adaptées à un usage ambulatoire.

De ce fait, de nombreux travaux ont été réalisés dans le but de développer des formes galéniques destinées à une administration *per* os qui, tout en se présentant sous une forme solide, sont aptes, lorsqu'elles sont placées dans la cavité buccale, à se déliter rapidement au contact de la salive pour former une suspension aisée à déglutir.

C'est ainsi que les Laboratoires PROGRAPHARM ont proposé, dans la Demande de Brevet Français n° 91 09245, des comprimés multiparticulaires dans lesquels le principe actif est présent sous la forme de microcristaux enrobés dans de l'éthylcellulose ou de microgranules, et est dispersé dans un excipient constitué par au moins deux agents désintégrants du type carboxyméthylcellulose sodique ou polyvinylpyrrolidone réticulée, par un ou plusieurs agents gonflants du type amidon, amidon modifié ou cellulose microcristalline, et par un sucre de compression directe.

Ultérieurement, ces mêmes Laboratoires, souhaitant améliorer la texture de ces comprimés, en raison de ce qu'ils génèrent en bouche une sensation sableuse et pâteuse, ont développé une technologie connue sous la marque FLASHTAB®, qui est décrite dans la Demande de Brevet Français n° 97 09233 et qui consiste à enrober des microcristaux de principe actif dans un polyméthacrylate ou un polymère cellulosique destiné à en masquer le goût, et à utiliser, comme excipient, un mélange d'un agent désintégrant (carboxyméthylcellulose sodique, polyvinylpyrrolidone réticulée) et d'un polyol à courte chaîne carbonée tel que le mannitol, le xylitol ou le sorbitol, ce polyol remplissant le rôle d'agent diluant à propriétés liantes.

La Demande de Brevet Français n° 88 15183 au nom de D. VACHER décrit des comprimés destinés à faciliter l'administration orale d'un principe actif médicamenteux principalement, mais non exclusivement, chez les enfants et, plus particulièrement, chez les nourrissons, et dans lesquels le principe actif représente au moins 60% en poids des comprimés, de manière à réduire au mieux leur taille, et est dispersé dans un excipient constitué par une gomme de cellulose réticulée et par un agent gonflant du type cellulose microcristalline ou amidon.

Constatant que l'usage de tels comprimés ne peut, en pratique, être envisagé chez les adultes, dans la mesure où ils ne peuvent contenir que de faibles doses de principe actif - ce qui impliquerait une multiplicité de prises -, D. VACHER a proposé, dans la Demande Internationale n° 96/02237, d'améliorer ces comprimés en enrobant le principe actif dans un agent liant hydrodispersible du type alcoylcellulose pour faciliter la mouillabilité de ce principe actif au contact de la salive, et en dispersant le principe actif ainsi enrobé dans un excipient constitué par de la carboxyméthylcellulose réticulée qui joue, dans ce cas, le rôle d'agent d'éclatement des comprimés, par de la cellulose microcristalline qui sert de diluant, et par un polyol soluble dans l'eau du type mannitol, xylitol ou sorbitol, qui est, lui, destiné à masquer le goût du principe actif.

Parallèlement, un certain nombre de recherches ont été consacrées au développement de formes galéniques aptes à se déliter en bouche par un processus d'effervescence maîtrisée.

Ainsi, par exemple, la Demande Internationale n° 91/04757 au nom de CIMA LABS décrit des comprimés dans lesquels le principe actif est dispersé, éventuellement sous une forme microéncapsulée, dans un mélange comprenant à la fois un acide du type acide citrique, acide tartrique, acide malique ou acide fumarique, et un carbonate tel que du bicarbonate de sodium, de manière à ce que cet acide et ce carbonate réagissent ensemble au contact de la salive pour libérer du gaz carbonique et permettre, ainsi, le délitement desdits comprimés.

De manière similaire, les Demandes de Brevets Français n° 94 01457 et 95 03711 au nom de X. HESNARD se rapportent à des comprimés dont le délitement en bouche est également obtenu par une réaction entre un acide et un carbonate, l'acide étant, dans ce cas, en quantité notablement supérieure à celle du carbonate de manière à provoquer, dès l'introduction des comprimés dans la cavité buccale, une hypersalivation propre à augmenter le volume hydrique intrabuccal, ce qui a pour effet de favoriser la désintégration desdits comprimés en une dispersion qui peut alors être avalée par une simple déglutition salivaire.

Par ailleurs, il s'avère que la mise au point d'une forme galénique apte à se déliter rapidement en bouche pose un certain nombre de problèmes spécifiques, notamment parce qu'elle doit satisfaire à des conditions dont certaines sont contradictoires entre elles. Ainsi, elle doit présenter une cohésion et une dureté suffisantes pour ne pas être altérée au cours des différentes étapes de fabrication, de conditionnement et de stockage, tout en étant capable de se désintégrer de manière quasi-instantanée au contact de la salive. Elle doit, par ailleurs, avoir et laisser en bouche un goût agréable, même lorsque le principe actif médicamenteux qu'elle renferme présente, lui, une amertume prononcée. Il est, de plus, souhaitable que, bien que ne répondant pas aux mêmes exigences que les formes galéniques conventionnelles, elle puisse toutefois être fabriquée selon les procédés et au moyen des mêmes équipements que ceux utilisés pour la fabrication de ces dernières, de manière à rendre son coût de production le plus satisfaisant possible.

Des solutions partielles à ces problèmes ont été proposées dans les Demandes de Brevets Européens n° 0 553 777, 0 636 364 et 0 745 382.

Ainsi, la Demande de Brevet Européen n° 0 553 777 au nom de TAKEDA CHEMICALS INDUSTRIES décrit un procédé qui consiste à mélanger le principe actif avec un excipient comprenant principalement un ou plusieurs carbohydrates du type sucrose, glucose, maltitol, xylitol et erythritol, puis à mouiller le mélange résultant par une faible quantité d'eau (de préférence, comprise entre 0,7 et 3%, p/p) et à le soumettre à une granulation et, enfin, après séchage des granules, à compresser ces derniers. Selon cette Demande, ce procédé présenterait le double avantage de conduire à l'obtention de comprimés dotés d'une dureté satisfaisante et de pouvoir être mis en oeuvre au moyen de granulateurs et de machines à compression habituellement utilisés dans l'industrie pharmaceutique.

La Demande de Brevet Européen n°0 636 364 au nom de McNEILL-PPC propose des comprimés dans lesquels le. principe actif se présente sous la forme de particules enrobées par un mélange de polymères cellulosiques destinés à en masquer le goût, et est dispersé sous cette forme dans un mélange comprenant un carbohydrate compressible apte à se désintégrer au contact de l'eau (mannitol, sorbitol, dextrose, saccharose, ...) et un liant du type cellulose et ses dérivés, polyvinylpyrrolidone, amidon ou cellulose microcristalline qui, lui, a vocation à conférer une cohésion satisfaisante aux comprimés. Selon cette Demande, l'enrobage du principe actif, puis la compression du mélange particules de principe actif enrobées/carbohydrate compressible/liant pourraient également être réalisées au moyen des équipements dont est traditionnellement équipée l'industrie pharmaceutique.

La Demande de Brevet Européen n° 0 745 382 au nom de YAMANOUCHI PHARMACEUTICAL décrit, quant à elle, des comprimés présentant une dureté propre à éviter qu'ils ne se cassent lors des opérations de fabrication, de conditionnement et de stockage, lesquels comprimés sont obtenus par un procédé consistant à soumettre à une granulation, séparément ou après les avoir mélangés, un principe actif, un saccharide à haute malléabilité du type maltose, maltitol ou sorbitol, et un saccharide à faible malléabilité comme le lactose, le mannitol ou le glucose, puis à compresser les granules résultants. Là encore, ce procédé est présenté comme étant susceptible d'être mis en oeuvre au moyen de granulateurs et de machines à compression traditionnelles.

En dépit de toutes ces tentatives, il s'avère qu'aucune forme galénique solide à délitement rapide en bouche ne donne totalement satisfaction à ce jour.

En effet, les comprimés utilisant la technologie FLASHTAB® décrite dans la Demande de Brevet Français n° 97 09233 procurent une sensation sableuse et pâteuse en bouche, même si celle-ci est théoriquement moins prononcée que celle observée avec des comprimés conformes à la Demande de Brevet Français n° 91 09245.

Il en est de même des comprimés effervescents proposés dans les Demandes de Brevets Français n° 91 04757, 94 01457 et 95 03711, ces comprimés présentant, en outre, une astringence marquée du fait de la quantité élevée d'acide qu'ils renferment.

L'enrobage du principe actif par des polymères destiné à en masquer le goût, comme décrit dans la Demande de Brevet Français n° 97 09233 et la Demande de Brevet Européen n° 0 636 364, se révèle être peu efficace, en sorte qu'il ne parvient pas à empêcher le développement d'une amertume en bouche, lorsque le principe actif est lui-même amer. Au surplus, cet enrobage complique notablement le procédé de préparation des comprimés.

Enfin, bon nombre des comprimés décrits dans les Demandes de Brevets ci-avant mentionnées (FR-A-97 09233, WO-A-96/02237, EP-A-0 636 364, EP-A-0 745 382) sont prévus pour renfermer des quantités importantes d'un polyol commé le mannitol, le xylitol et le sorbitol, dont les effets laxatifs bien connus s'opposent à une administration répétée de ce type de comprimés et, partant, à leur utilisation pour des traitements au long cours.

Il a été également proposé, dans les Demandes Internationales n° 97/28788 et 97/28789, de réaliser des comprimés aptes à se dissoudre rapidement en milieu aqueux ― mais dont la dissolution n'est toutefois pas spécialement destinée à se faire en bouche ― à partir de mélanges comprenant, outre un principe actif, du tréhalose en tant qu'agent diluant, un agent liant en quantité suffisante pour conférer aux comprimés une dureté satisfaisante, lequel peut notamment être de la copovidone, et un sel volatile du type bicarbonate ou acétate d'ammonium, qui est éliminé après les opérations de compression et dont la fonction est de conférer aux comprimés une porosité propice à leur dissolution au contact d'un milieu aqueux.

Tout en envisageant la possibilité que le tréhalose présent dans le mélange soit aussi bien sous forme dihydratée cristalline, amorphe qu'anhydre, ces Demandes enseignent qu'en fait, seul le tréhalose anhydre est à même, en raison de sa capacité à absorber l'humidité, de garantir une stabilité du principe actif et autorise l'utilisation conjointe d'un sel volatile. Or, il s'avère que le tréhalose anhydre n'est pas disponible commercialement en sorte que la réalisation de comprimés tels que proposés dans WO-A-97/28788 et WO-A-97/28789 nécessite de préparer préalablement ce composé avec tous les inconvénients que cela implique comme la nécessité de disposer d'équipements particuliers et l'augmentation du coût de fabrication des comprimés. L'utilisation d'un sel volatile complique, elle aussi, notablement le procédé de fabrication des comprimés et en grève sérieusement le coût, puisque ce sel doit être éliminé à l'issue des opérations de compression, en plaçant les comprimés sous vide et à une température de 60°C pendant plusieurs heures.

Enfin, on connaît, par la Demande de Brevet Européen n° 0 376 891, des comprimés à base de baclofène qui sont conçus pour adhérer à l'une des muqueuses buccales et, notamment, à la muqueuse palatine afin d'assurer une libération à la fois immédiate et prolongée du baclofène. Ces comprimés sont constitués par un coeur hydrophile dans lequel se trouve le baclofène, un polymère vinylique, un galactomannane (gomme guar), une cire ou un glycéride, et par une pellicule hydrophobe qui recouvre le coeur hydrophile sauf au niveau d'une petite surface qui est celle destinée à adhérer à la muqueuse. Comme l'illustrent les exemples de cette Demande, les comprimés se dissolvent en bouche en 12 à 15 heures. Ils ne sauraient donc être considérés comme des comprimés à délitement rapide.

La Demanderesse s'est donc fixé pour but de fournir une forme galénique solide qui, tout en étant apte à se déliter rapidement et complètement en bouche de manière à limiter au maximum l'effort de déglutition nécessaire à l'ingestion d'un principe actif, ait une texture agréable et un goût acceptable y compris dans le cas ou le principe actif présente une amertume prononcée, présente une cohésion et une dureté suffisamment élevées pour éviter qu'elle ne s'altère au cours des étapes de fabrication, de conditionnement et de stockage, et puisse être fabriquée par un procédé très simple de manière à en limiter le coût de production.

Ce but est atteint selon la présente Invention par une forme galénique du type comprenant au moins un principe actif dispersé dans un mélange d'excipients et qui est caractérisée en ce que le mélange d'excipients comprend au moins un agent diluant faiblement compressible différent du tréhalose et un copolymère de 1-vinylpyrrolidin-2-one et d'acétate de vinyle.

La Demanderesse a, en effet, trouvé qu'en dispersant un principe actif dans un mélange comprenant à la fois un agent diluant faiblement compressible ― dont l'utilisation est, de ce fait, préconisée pour réaliser des formes galéniques nécessitant une granulation par voie humide ― et un copolymère de 1-vinylpyrrolidin-2-one et d'acétate de vinyle, il est possible d'obtenir, par une simple opération de compression, une forme galénique qui, de façon surprenante, est non seulement apte à se déliter complètement en un temps extrêmement court, de l'ordre de 30 à 50 secondes dans la cavité buccale, mais présente de plus :
- un fondant en bouche très agréable,
- un goût tout à fait convenable, même en cas d'amertume prononcée du principe actif, et
- une cohésion et une dureté satisfaisantes et ce, en dépit de la faible compressibilité de l'agent diluant présent dans cette forme galénique.

L'agent diluant faiblement compressible est choisi parmi les dextrates, tels que l'Emdex^{(R)} de la Société MENDELL, et le D-glucose monohydraté. L'agent diluant faiblement compressible joue un rôle de remplissage dans le but d'obtenir un comprimé de caractéristiques et de taille convenables, et qui présente une dureté comprise entre 40 et 50 N lorsqu'on applique une force de 25 kg/cm² à un comprimé constitué par 500 mg de cette substance et mesurant 20 mm de diamètre sur 4,5 mm d'épaisseur.

De préférence, l'agent diluant faiblement compressible est du D-glucose monohydraté, qui présente le double avantage d'être intéressant d'un point de vue économique et d'être acariogène. Ce composé est disponible par exemple, auprès de la Société ROQUETTE sous la marque Roférose®.

Selon une autre disposition avantageuse de l'Invention, le copolymère de 1-vinylpyrrolidin-2-one et d'acétate de vinyle est de la copovidone. Cette dernière, qui est classiquement utilisée en galénique pour ses propriétés liantes et désintégrantes, est notamment commercialisée par la Société BASF sous la marque Kollidon® VA 64.

Conformément à l'Invention, l'agent diluant faiblement compressible et le copolymère de 1-vinylpyrrolidin-2-one et d'acétate de vinyle sont présents dans la forme galénique dans un rapport en poids avantageusement compris entre 1,5 et 30. De manière préférée, ce rapport est compris entre 3 et 22.

Selon encore une autre disposition avantageuse de l'Invention, l'agent diluant faiblement compressible et le copolymère de 1-vinylpyrrolidin-2-one et d'acétate de vinyle représentent ensemble entre 25 et 65% en poids du poids total de la forme galénique.

Comme précédemment mentionné, la présence du copolymère de 1-vinylpyrrolidin-2-one et d'acétate de vinyle dans le mélange d'excipients permet de conférer à ce dernier une compressibilité suffisamment élevée pour obtenir une forme galénique de dureté satisfaisante. Toutefois, dans un certain nombre de cas et, notamment, lorsque la forme galénique est destinée à renfermer une quantité de principe actif représentant plus de 25 % en poids du poids total de la forme galénique, il est possible de prévoir que le mélange d'excipients comprenne de plus un polyol propre à augmenter encore sa compressibilité.

Conformément à l'Invention, ce polyol est avantageusement choisi parmi les glucitols et les diglucitols comme le mannitol, le sorbitol, le xylitol et le lactitol, et représente au plus 15% en poids du poids total de la forme galénique.

De manière préférée, le polyol est du mannitol granulé (tel que celui commercialisé par la Société ROQUETTE sous la marque Pearlitol®) pour un principe actif sensible à l'eau (le mannitol est peu hygroscopique), ou du sorbitol granulé (tel que celui commercialisé par la Société ROQUETTE sous la marque Néosorb®) qui a aussi une compressibilité élevée.

La forme galénique conforme à l'Invention, peut comprendre encore d'autres excipients et notamment des excipients choisis parmi les agents de remplissage, les agents d'écoulement, les agents lubrifiants, les agents de sapidité, les agents édulcorants et les arômes. Ainsi, la forme galénique conforme à l'Invention peut comprendre :
- un ou plusieurs agents de remplissage choisis, de préférence, parmi la cellulose microcristalline, l'amidon prégélatinisé et la carboxyméthylcellulose ; ces agents de remplissage, qui ont pour fonction d'accélérer la désagrégation de la forme galénique en bouche sans en altérer la dureté, sont avantageusement présents dans des proportions comprises entre 10 et 30% en poids du poids total de la forme galénique ;
- un ou plusieurs agents d'écoulement choisis, de préférence, parmi la silice colloïdale anhydre, le talc et l'acide stéarique ; ces agents d'écoulement, qui sont destinés d'une part, à éviter que les composants de la forme galénique ne forment des agglomérats au cours de sa préparation, et, d'autre part, à réduire les effets de friction pendant la compression, sont avantageusement présents dans des proportions comprises entre 0,1 et 0,5% en poids du poids total de ladite forme galénique ;
- un ou plusieurs agents lubrifiants choisis, de préférence, parmi le stéarate de magnésium, le stéarate de calcium, l'acide stéarique et le dibéhénate de glycérol (Compritol® - Société GATTEFOSSE) ; ces agents lubrifiants, qui sont également destinés à réduire les effets de frictions pendant la compression, sont avantageusement présents dans des proportions comprises entre 0,5 et 5 % en poids du poids total de la forme galénique ;
- un ou plusieurs agents de sapidité tels que l'acide citrique ou le citrate de sodium ; ces agents de sapidité sont avantageusement présents dans des proportions avantageusement comprises entre 0,5 et 5 % en poids du poids total de la forme galénique;
- un ou plusieurs agents édulcorants comme l'aspartame, la saccharine sodique, le cyclamate ou l'acésulfame de potassium, et/ou un ou plusieurs arômes tels que menthe, framboise, réglisse, orange, citron ou fraise ; ces agents édulcorants et ces arômes, dont le rôle est de potentialiser les effets de l'agent ou des agents de sapidité et, partant, de conférer à la forme galénique un goût convenable, représentent avantageusement entre 0,5 et 5 % en poids du poids total de la forme galénique.

La forme galénique conforme à l'Invention peut être utilisée pour l'administration par voie orale de très nombreux principes actifs. A titre d'exemples et de façon non-limitative, on peut citer les antalgiques comme l'aspirine, le paracétamol, l'ibuprofène, le tramadol, la codéine, le dextropropoxyfène, la buprénorphine, le bénorilate et la morphine ; les agents antispasmodiques comme le phloroglucinol ; les agents utilisés en gastro-entérologie comme le cisapride, le dompéridone et la métopimazine ; les antinaupathiquès comme le dimenhydrinate ; les agents antimigraineux comme la dihydroergotamine et le sumatriptan ; les β-bloquants comme le céliprolol et le bisopropol ; les agents antihistaminiques comme la loratadine, la cétirizine et le kétotifen ; les agents antibiotiques ou antibactériens du type β-lactamines, macrolides, quinolones ou céphalosporines ; les agents antivertigineux comme la bétahistine et les agents hypnotiques comme la zopiclone, le lorazépam, le bromazépam, l'alprazolam et la doxylamine, tous ces principes actifs étant susceptibles d'être présents seuls ou en association.

Dans la mesure où la forme galénique conforme à l'Invention présente un goût tout à fait convenable et ce, quel que soit le goût du principe actif lui-même, cette forme est. de préférence, un comprimé nu. Toutefois, on peut très bien envisager qu'elle soit recouverte d'une fine pellicule, par exemple de sucre, destinée à rendre encore plus agréable son contact avec la cavité buccale et. notamment, avec la langue.

La présente Invention a, également, pour objet un procédé de préparation d'une forme galénique conforme à l'Invention, lequel procédé est caractérisé en ce qu'il comprend :
- le mélange du ou des principes actifs avec l'agent diluant faiblement compressible, le copolymère de 1-vinylpyrrolidin-2-one, et, le cas échéant, le ou les agents de remplissage, agents d'écoulement, agents de sapidité, agents édulcorants et arômes,
- l'incorporation du ou des agents lubrifiants, si l'on souhaite utiliser ce type d'agents, et
- la compression du mélange résultant.

Cette compression peut-être réalisée selon les mêmes techniques et au moyen du même matériel que ceux utilisés pour la préparation de comprimés et de tablettes conventionnels.

La forme galénique conforme à l'Invention présente de nombreux avantages. En effet, outre de présenter un délitement en bouche, une texture, un goût et une dureté très satisfaisantes (cette dernière étant généralement comprise entre 70 et 150 N), elle se caractérise par l'absence ou la présence en très faible quantité d'un polyol, en sorte qu'elle réduit notablement les risques d'effets secondaires liés à l'ingestion de ce type de composés, notamment dans le cadre de traitement au long cours. Elle est, de plus, susceptible d'être obtenue par un procédé qui est simple à mettre en oeuvre, notamment parce qu'il ne nécessite aucune étape d'enrobage du principe actif, et qui est, de ce fait, économiquement très attractif.

Aussi, est-elle susceptible d'être utilisée dans de très nombreuses indications thérapeutiques et ce, aussi bien chez l'adulte que chez l'enfant. Son utilisation présente un intérêt tout particulier dans le traitement de symptômes ou de pathologies nécessitant un soulagement rapide. A titre d'exemples et de manière non-limitative, on peut citer les céphalées, les migraines, les spasmes digestifs, les vertiges, les nausées, les vomissements, les réactions allergiques, l'hypertension, l'insuffisance coronarienne et les insomnies.

Outre les dispositions qui précèdent, l'Invention comprend encore d'autres dispositions qui ressortiront du complément de description qui suit et qui se rapporte à des exemples de réalisation de formes galéniques conformes à l'Invention.

Il doit, toutefois, être bien entendu que ces exemples sont donnés à titre d'illustrations de l'objet de l'Invention et n'en constituent en aucune manière une limitation.

### EXEMPLE 1 : Préparation de comprimés nus contenant 500 mg de paracétamol

On prépare des comprimés présentant chacun la composition qualitative et quantitative ci-après :

| | |
|---|---|
| Paracétamol cristallisé | 500,0 mg |
| D-glucose monohydraté | 597,6 mg |
| Copovidone | 35,2 mg |
| Cellulose microcristalline | 160,0 mg |
| Acide citrique anhydre | 35,2 mg |
| Sorbitol granulé | 160,0 mg |
| Aspartam | 28,8 mg |
| Saccharine sodique | 14,4 mg |
| Dibéhénate de glycérol | 16,0 mg |
| Stéarate de magnésium | - 6,4 mg |
| Arôme orange | 46,4 mg |

en opérant de la manière suivante :
- on mélange, au moyen d'un mélangeur à retournement, tous les composants à l'exception des agents lubrifiants (stéarate de magnésium et dibéhénate de glycérol), jusqu'à l'obtention d'un ensemble homogène,
- on ajoute le stéarate de magnésium et le dibéhénate de glycérol, et on mélange à nouveau jusqu'à homogénéité, puis
- on soumet le mélange résultant à une compression pour obtenir des comprimés présentant un poids unitaire de 1,6 g et mesurant 20 mm de diamètre sur 4,5 mm d'épaisseur.

Les comprimés ainsi préparés se délitent en bouche en 30 à 45 secondes.

### EXEMPLE 2 : Préparation de comprimés nus contenant 50 mg de chlorhydrate de tramadol

On prépare des comprimés présentant chacun la composition qualitative et quantitative ci-après:

| | |
|---|---|
| Chlorhydrate de tramadol | 50,0 mg |
| D-glucose monohydraté | 225,0 mg |
| Copovidone | 11,0 mg |
| Cellulose microcristalline | 175,0 mg |
| Acide citrique anhydre | 11,0 mg |
| Aspartam | 7,5 mg |
| Saccharine sodique | 3,5 mg |
| Dibéhénate de glycérol | 5,0 mg |
| Acide stéarique | 2,0 mg |
| Arôme framboise | 10,0 mg |

en suivant un protocole opératoire similaire à celui décrit dans l'exemple 1, à ceci près que la compression est réalisée de manière à obtenir des comprimés présentant un poids unitaire de 500 mg et mesurant 12 mm de diamètre sur 4 mm d'épaisseur.

Les comprimés ainsi préparés se délitent en bouche en 30 à 40 secondes.

### EXEMPLE 3 : Préparation de comprimés nus contenant 200 mg d'ibuprofène

On prépare des comprimés présentant chacun la composition qualitative et quantitative ci-après :

| | |
|---|---|
| Ibuprofène | 200,0 mg |
| D-glucose monohydraté | 378,0 mg |
| Copovidone | 80,0 mg |
| Cellulose microcristalline | 180,0 mg |
| Acide citrique anhydre | 20,0 mg |
| Mannitol granulé | 80,0 mg |
| Aspartam | 20,0 mg |
| Saccharine sodique | 10,0 mg |
| Silice colloïdale anhydre | 2,0 mg |
| Stéarate de magnésium | 10,0 mg |
| Arôme menthe | 20,0 mg |

en suivant un protocole opératoire similaire à celui décrit dans l'exemple 1, à ceci près que la compression est réalisée de manière à obtenir des comprimés présentant un poids unitaire de 1 g et mesurant 16 mm de diamètre sur 5 mm d'épaisseur.

Les comprimés ainsi préparés se délitent en bouche en 30 à 50 secondes.

## Revendications

1. Forme galénique à délitement rapide en bouche, du type comprenant au moins un principe actif dispersé dans un mélange d'excipients, **caractérisée en ce que** le mélange d'excipients comprend au moins un agent diluant faiblement compressible choisi parmi les dextrates et le D-glucose monohydraté et un copolymère de 1-vinylpyrrolidin-2-one et d'acétate de vinyle.

2. Forme galénique selon la revendication 1, **caractérisée en ce que** le copolymère de 1-vinylpyrrolidin-2-one et d'acétate de vinyle est de la copovidone.

3. Forme galénique selon la revendication 1 ou 2, **caractérisée en ce que** l'agent diluant faiblement compressible et le copolymère de 1-vinylpyrrolidin-2-one et d'acétate de vinyle sont présents dans un rapport en poids compris entre 1,5 et 30 et, de préférence, entre 3 et 22.

4. Forme galénique selon l'une quelconque des revendications précédentes, **caractérisée en ce que** l'agent diluant faiblement compressible et le copolymère de 1-vinylpyrrolidin-2-one et d'acétate de vinyle représentent ensemble entre 25 et 65% en poids du poids total de la forme galénique.

5. Forme galénique selon l'une quelconque des revendications précédentes, **caractérisée en ce qu'**elle comprend un polyol.

6. Forme galénique selon la revendication 5, **caractérisée en ce que** le polyol est choisi parmi les glucitols et les diglucitols, et représente au plus 15% en poids du poids total de ladite forme galénique.

7. Forme galénique selon la revendication 6, **caractérisée en ce que** le polyol est du mannitol granulé ou du sorbitol granulé.

8. Forme galénique selon l'une quelconque des revendications précédentes, **caractérisée en ce que** le mélange d'excipients comprend un ou plusieurs agents de remplissage, agents d'écoulement, agents lubrifiants, agents de sapidité, agents édulcorants et un ou plusieurs arômes.

9. Forme galénique selon la revendication 8, **caractérisée en ce que** le(s) agent(s) de remplissage est(sont) choisi(s) parmi la cellulose microcristalline, l'amidon prégélatinisé et la carboxyméthylcellulose, et est(sont) présent(s) dans des proportions comprises entre 10 et 30 % en poids du poids total de ladite forme galénique.

10. Forme galénique selon la revendication 8, **caractérisée en ce que** le(s) agent(s) d'écoulement est(sont) choisi(s) parmi la silice colloïdale anhydre, le talc et l'acide stéarique, et est(sont) présent(s) dans des proportions comprises entre 0,1 et 0,5 % en poids du poids total de ladite forme galénique.

11. Forme galénique selon la revendication 8, **caractérisée en ce que** le(s) agent(s) lubrifiant(s) est(sont) choisi(s) parmi le stéarate de magnésium, le stéarate de calcium, l'acide stéarique et le dibéhénate de glycérol, et est(sont) présent(s) dans des proportions comprises entre 0,5 et 2,5 % en poids du poids total de ladite forme galénique.

12. Forme galénique selon la revendication 8, **caractérisée en ce que** le(s) agent(s) de sapidité est(sont) choisi(s) parmi l'acide citrique et le citrate de sodium, et est(sont) présent(s) dans des proportions comprises entre 0,5 et 5 % en poids du poids total de ladite forme galénique.

13. Forme galénique selon la revendication 8, **caractérisée en ce que** le(s) agent(s) édulcorant(s) et l'(es) arôme(s) représentent avantageusement entre 0,5 et 5 % en poids du poids total de ladite forme galénique.

14. Forme galénique selon l'une quelconque des revendications précédentes, **caractérisée en ce que** le principe actif est choisi parmi les antalgiques, les agents antispasmodiques, les agents utilisés en gastro-entérologie, les agents antinaupathiques, les agents antimigraineux, les agents β-bloquants, les agents antihistaminiques, les agents antibiotiques ou antibactériens, les agents antivertigineux et les agents hypnotiques, seuls ou en association.

15. Forme galénique selon l'une quelconque des revendications précédentes, **caractérisée en ce que** le principe actif est du paracétamol ou du tramadol.

16. Forme galénique selon l'une quelconque des revendications précédentes, **caractérisée en ce qu'**elle est un comprimé nu.

17. Procédé de préparation d'une forme galénique selon l'une quelconque des revendications 1 à 16, **caractérisé en ce qu'**il comprend :
- le mélange du ou des principes actifs avec l'agent diluant faiblement compressible, le copolymère de 1-vinylpyrrolidin-2-one, et, le cas échéant, le ou les agents de remplissage, agents d'écoulement, agents de sapidité, agents édulcorants et arômes,
- l'incorporation du ou des agents lubrifiants, si l'on souhaite utiliser ce type d'agents; et
- la compression du mélange résultant.

## Patentansprüche

1. Galenische Form für den schnellen Zerfall im Mund, umfassend mindestens ein aktives Prinzip, das in einem Exzipienziengemisch verteilt ist, **dadurch gekennzeichnet, dass** das Exzipienziengemisch mindestens ein schwach komprimierbares Verdünnungsmittel ausgewählt aus Dextraten und D-Glukose-Monohydrat und ein Copolymer aus 1-Vinylpyrrolidin-2-on und Vinylacetat umfasst.

2. Galenische Form nach Anspruch 1, **dadurch gekennzeichnet, dass** das Copolymer aus 1-Vinylpyrrolidin-2-on und Vinylacetat Copovidon ist.

3. Galenische Form nach Anspruch 1 oder 2, **dadurch gekennzeichnet, dass** das schwach komprimierbare Verdünnungsmittel und das Copolymer aus 1-Vinylpyrrolidin-2-on und Vinylacetat in einem Gewichtsverhältnis zwischen 1,5 und 30 und bevorzugt zwischen 3 und 22 vorliegen.

4. Galenische Form nach einem der vorstehenden Ansprüche, **dadurch gekennzeichnet, dass** das schwach komprimierbare Verdünnungsmittel und das Copolymer aus 1-Vinylpyrrolidin-2-on und Vinylacetat zusammen zwischen 25 und 65% (Gew./Gew.) der gesamten galenischen Form einnehmen.

5. Galenische Form nach einem der vorstehenden Ansprüche, **dadurch gekennzeichnet, dass** sie ein Polyol umfasst.

6. Galenische Form nach Anspruch 5, **dadurch gekennzeichnet, dass** das Polyol ausgewählt ist aus Glucitolen und Diglucitolen, und mehr als 15% (Gew./Gew.) der gesamten galenischen Form einnimmt.

7. Galenische Form nach Anspruch 6, **dadurch gekennzeichnet, dass** das Polyol granuliertes Mannitol oder granuliertes Sorbitol ist.

8. Galenische Form nach einem der vorstehenden Ansprüche, **dadurch gekennzeichnet, dass** das Exzipienziengemisch eines oder mehrere Füllmittel, Fließmittel, Geschmacksmittel, Süßstoffe und eines oder mehrere Aromen umfasst.

9. Galenische Form nach Anspruch 8, **dadurch gekennzeichnet, dass** das/die Füllmittel ausgewählt ist/sind aus mikrokristalliner Cellulose, vorgelierter Stärke und Carboxymethylcellulose, und dass es/sie mit einem Anteil zwischen 10 und 30% (Gew./Gew.) der gesamten galenischen Form vorliegt/en.

10. Galenische Form nach Anspruch 8, **dadurch gekennzeichnet, dass** das/die Fließmittel ausgewählt ist/sind aus colloidalem wasserfreien Kieselsäureanhydrid, Talk und Stearinsäure, und dass es/sie mit einem Anteil zwischen 0,1 und 0,5% (Gew./Gew.) der gesamten galenischen Form vorliegt/en.

11. Galenische Form nach Anspruch 8, **dadurch gekennzeichnet, dass** das/die Trennmittel ausgewählt ist/sind aus Magnesiumstearat, Calciumstearat, Stearinsäure und Glyceryl-Dibehenat, und es/sie mit einem Anteil zwischen 0,5 und 25% (Gew./Gew.) der gesamten galenischen Form vorliegt/en.

12. Galenische Form nach Anspruch 8, **dadurch gekennzeichnet, dass** der/die Geschmacksstoff (e) ausgewählt ist/sind aus Zitronensäure und Natriumcitrat, und er/sie mit einem Anteil zwischen 0,5 und 5% (Gew./Gew.) der gesamten galenischen Form vorliegt/en.

13. Galenische Form nach Anspruch 8, **dadurch gekennzeichnet, dass** der/die Süßstoff (e) und der/die Aromastoff(e) vorteilhafterweise zwischen 0,5 und 5% (Gew./Gew.) der gesamten galenischen Form einnehmen.

14. Galenische Form nach einem der vorstehenden Ansprüche, **dadurch gekennzeichnet, dass** das aktive Prinzip ausgewählt ist aus Analgetika, Antispastika, Mitteln, die in der Gastroenterologie verwendet werden, Mitteln gegen Seekrankheit, Antimigränemitteln, Beta-Blockern, Antihistaminika, antibiotischen oder antibakteriellen Mitteln, antivertigenösen Mitteln und Hypnotika, und zwar alleine oder in Kombination.

15. Galenische Form nach einem der vorstehenden Ansprüche, **dadurch gekennzeichnet, dass** das aktive Prinzip Paracetamol oder Tramadol ist.

16. Galenische Form nach einem der vorstehenden Ansprüche, **dadurch gekennzeichnet, dass** sie eine unverhüllte Tablette ist.

17. Verfahren zur Herstellung einer galenischen Form nach einem der Ansprüche 1 bis 16, **dadurch gekennzeichnet, dass** es umfasst:
- Mischen des aktiven Prinzips oder der aktiven Prinzipien mit dem schwach komprimierbaren Verdünnungsmittel, dem Copolymer aus 1-Vinylpyrrolidin-2-on und gegebenenfalls des/der Füllmittel, Fließmittel, Geschmacksstoffe, Süßstoffe und Aromen,
- Einbringen des/der Trennmittel(s), wenn man diese Art Mittel verwenden möchte, und
- Pressen des daraus hervorgehenden Gemisches.

## Claims

1. Galenic form that rapidly dissolves in the mouth, of the type which consists of at least one active ingredient in a mixture of excipients, it is **characterised by** the fact that the mixture of excipients has at least one weakly compressible diluting agent chosen among dextrates and D-glucose monohydrate and one copolymer of 1-vinylpyrrolidin-2-one and vinyl acetate.

2. Galenic form according to claim 1, **characterised by** the fact that the copolymer of 1 -vinylpyrrolidin-2-one and vinyl acetate is copovidone.

3. Galenic form according to claim 1 or 2, **characterised by** the fact that the weakly compressible diluting agent and the copolymer of 1-vinylpyrrolidin-2-one and vinyl acetate are present in a weight ratio of between 1.5 and 30, preferably between 3 and 22.

4. Galenic form according to any of the aforementioned claims, **characterised by** the fact that the weakly compressible diluting agent and the copolymer of 1-vinylpyrrolidin-2-one and vinyl acetate represent, together, between 25 and 65% in weight of the galenic form's total weight.

5. Galenic form according to any of the aforementioned claims, **characterised by** the fact that it includes a polyol.

6. Galenic form according to claim 5, **characterised by** the fact that the polyol is chosen among glucitols and diglucitols, and represents a maximum of 15% in weight of the aforesaid galenic form' s total weight.

7. Galenic form according to claim 6, **characterised by** the fact that the polyol is granulated mannitol or granulated sorbitol.

8. Galenic form according to any of the aforementioned claims, **characterised by** the fact that the mixture of excipients includes one or more fillers, flow agents, lubricating agents, palatabiliting agents, sweetening agents and one or more flavourings.

9. Galenic form according to claim 8, **characterised by** the fact that the filler(s) is(are) chosen among microcrystalline cellulose, pregelatinised starch and carboxymethylcellulose, and is(are) present in ratios of between 10 and 30% in weight of the aforesaid galenic form's total weight.

10. Galenic form according to claim 8, **characterised by** the fact that the flow agent(s) is(are) chosen among colloidal anhydrous silica, talcum and stearic acid, and is(are) present in ratios of between 0.1 and 0.5% in weight of the aforesaid galenic form's total weight.

11. Galenic form according to claim 8, **characterised by** the fact that the lubricating agent(s) is(are) chosen among magnesium stearate, calcium stearate, stearic acid and glycerol dibehenate, and is(are) present in ratios of between 0.5 and 2.5% in weight of the aforesaid galenic form's total weight.

12. Galenic form according to claim 8, **characterised by** the fact that the palatabiliting agent(s) is(are) chosen among citric acid and sodium citrate, and is(are) present in ratios of between 0.5 and 5% in weight of the aforesaid galenic form's total weight.

13. Galenic form according to claim 8, **characterised by** the fact that the sweetening agent(s) and the flavouring(s) represent, favourably, between 0.5 and 5% in weight of the aforesaid galenic form's total weight.

14. Galenic form according to any of the aforementioned claims, **characterised by** the fact that the active ingredient is chosen among analgesics, antispasmodic agents, agents used in gastroenterology, seasickness remedy agents, antimigraine agents, β-blocking agents, antihistamine agents, antibiotic or antibacterial agents, anti-vertigo agents and hypnotic agents, alone or combined.

15. Galenic form according to any of the aforementioned claims, **characterised by** the fact that the active ingredient is paracetamol or tramadol.

16. Galenic form according to any of the aforementioned claims, **characterised by** the fact that it is an uncoated tablet.

17. Preparation technique of a galenic form according to any of the aforementioned claims 1 to 16, **characterised by** the fact that:
- the blending of the active ingredient or ingredients with the weakly compressible diluting agent, the copolymer of I -vinylpyrrolidin-2-one, and, where appropriate, the filler or fillers, flow agents, flavouring agents, sweetening agents and flavourings.
- adding (a) lubricating agent or agents, if one desires to use this kind of agent, and
- compacting the resulting mixture.
